# EUROPÄISCHE PATENTSCHRIFT

(11) **EP 1 785 665 B1**
(45) Veröffentlichungstag und Bekanntmachung des Hinweises auf die Patenterteilung: **02.11.2011**
(21) Anmeldenummer: 05024819.4
(22) Anmeldetag: 14.11.2005
(51) Int. Cl.: F21S 8/00, H04N 9/73, H05B 33/08

(54) **Operationsleuchte**
Surgical lamp
Lampe chirurgicale

(43) Veröffentlichungstag der Anmeldung: 16.05.2007
(73) Patentinhaber: TRUMPF Medizin Systeme GmbH + Co. KG, 07318 Saalfeld (DE)
(72) Erfinder: Marka, Rudolf Dr., 81479 München (DE); Vogel, Markus, 82008 Unterhaching (DE)
(74) Vertreter: Feldmeier, Jürgen

(56) Entgegenhaltungen:
- WO-A-99/22224
- US-A1- 2004 129 860
- US-A1- 2005 195 599

## Beschreibung

Die Erfindung betrifft eine Operationsleuchte mit mindestens einem weißen und mehreren farbigen Leuchtmitteln zur Ausleuchtung der Operationsstelle und mit einer Steuerung für die Leuchtmittel.

Derartige Operationsleuchten sind durch ihre Verwendung in Operationssälen allgemein bekannt geworden.

Wünschenswert ist es, bei einer Operationsleuchte deren Lichtstrom bezüglich der Farbtemperatur, der Intensität und der Verteilung über die Lichtaustrittsfläche zu steuern. Diese Steuerung ist bei Operationsleuchten mit herkömmlichen Leuchtmitteln, wie Halogen-oder Gasentladungsleuchten, nicht oder nur sehr aufwändig möglich. Die Farbtemperatur von Halogen- oder Gasentladungsleuchtmitteln ist bei der Verwendung von einzelnen Leuchtmitteln nicht während des Betriebs gezielt einstellbar, sondern nur durch den Einsatz von Filtertechnik veränderbar. Bei einem Einsatz von mehreren teueren Leuchtmitteln kann die Farbtemperatur in bestimmten Grenzen verändert werden. Die Helligkeit ist durch die Verwendung von Blenden ohne Veränderung der Farbtemperatur einstellbar. Bei einer elektrischen Dimmung verändert sich mit der Helligkeit die Farbtemperatur. Eine Veränderung der Verteilung ist nur grob, durch Blendentechnik oder den Einsatz von mehreren teuren Leuchtmitteln, möglich.

Die Offenlegung US-A-2004/0129860 beschreibt eine Beleuchtungsvorrichtung zur Erzeugung eines Leuchtfelds, im Speziellen ein Leuchtfeld für chirurgische Operationen, die verschiedenfarbige LEDs innerhalb eines Moduls aufweist und die einen Speicher zum Abspeichern eines Programms und von Betriebsparametern aufweist. Die LEDs sind einzeln mit einem Stromregler verbunden und separat ansteuerbar.

In der US-A-2005/0195599 ist eine Operationsleuchte beschrieben, die aus mehreren. Modulen besteht, welche eine Mehrzahl von verschiedenfarbigen LEDs enthalten. Die Intensität der Leuchtstärke der farbigen LEDs kann wahlweise verändert werden und durch die Mischung mit weißem Licht kann eine vom Operateur gewünschte Farbtemperatur des abgegebenen Lichts eingestellt werden. Die gewünschten. Sollwerte können in einem Speicherbereich der Steuerung abgespeichert werden.

Der Erfindung liegt die Aufgabe zu Grunde, die Ansteuerung der Leuchtmittel bezüglich der Einstellung der Farbtemperatur und der Intensität (Helligkeit) der Operationsteuchte zu verbessern.

Die Aufgabe wird mit den Merkmalen des Anspruchs 1 gelöst. Weiterbildungen der Erfindung sind Gegenstand der Unteransprüche.

Operationsleuchten sollen mit einer einstellbaren Farbtemperatur und einer einstellbaren Intensität (Helligkeit) ausgestattet sein. Dazu werden beispielsweise verschiedenfarbige LEDs (z.B. kaltweiß, warmweiß, cyan, blau) als Leuchtmittel verwendet, die dann mit einstellbaren Leistungswerten (d.h. beispielsweise Strom und/oder Spannung für eine Gruppe von Leuchtmitteln) angesteuert werden.

Um die Beleuchtungsparameter für einzelne Gruppen von Leuchtmitteln zu standardisieren, werden für die verschiedenen Beleuchtungsparameter die Leistungswerte den Gruppen zugeordnet abgespeichert.

Als Sollwerte für die Beleuchtungsparameter kommen vorzugsweise die Farbtemperatur der Operationsleuchte, die Leuchtintensität der Operationsleuchte oder die Verteilung der Lichtintensität über die Austrittsfläche in Betracht. Gruppen von Leuchtmitteln lassen sich mithilfe der Sollwerte ansteuern, regeln und kalibrieren.

Als ein mithilfe der Sollwerte regelbarer Leistungswert oder Lichtstromwert kommt vorzugsweise die leicht regelbare Stromstärke in Betracht. Der Stromwert kann eine Regelung der Stromstärke oder eine Impulsfolge für die Pulsweitenmodulation sein, um die Ansteuerung zu optimieren.

Vorteilhafterweise sind die Leuchtmittel zu einem oder mehreren Modulen oder Kombinationen zusammengefasst. Hierdurch wird die Möglichkeit geschaffen, unterschiedliche Operationsleuchten aufzubauen.

Jedem Modul oder jeder Kombination kann ein Mittel zur Speicherung (EPROM) zugeordnet sein. Weiterhin kann eine Datenübertragung zur zentralen Steuerung der Operationsleuchte ausgebildet sein. Dies hat den Vorteil, dass der Austausch von Modulen im Reparaturfall erleichtert wird. Mithilfe der Speicherung von Daten für jedes Modul ist es möglich, ein Austauschmodul mit denselben lichttechnischen Eigenschaften einzusetzen. Auch bei einem Austausch der Zentralsteuerung ist gewährleistet, dass die Leuchte mit ihren kalibrierten Werten betrieben wird.

Ein bevorzugtes Ausführungsbeispiel der Erfindung ist in der schematischen Zeichnung dargestellt und wird anhand der Zeichnung erläutert. Es zeigt:
- Figur 1: den näheren Aufbau eines Leuchtmoduls einer Operationsleuchte;
- Figur 2: die Operationsleuchte;
- Figur 3: ein Bedienfeld einer Bedieneinrichtung für die Operationsleuchte.

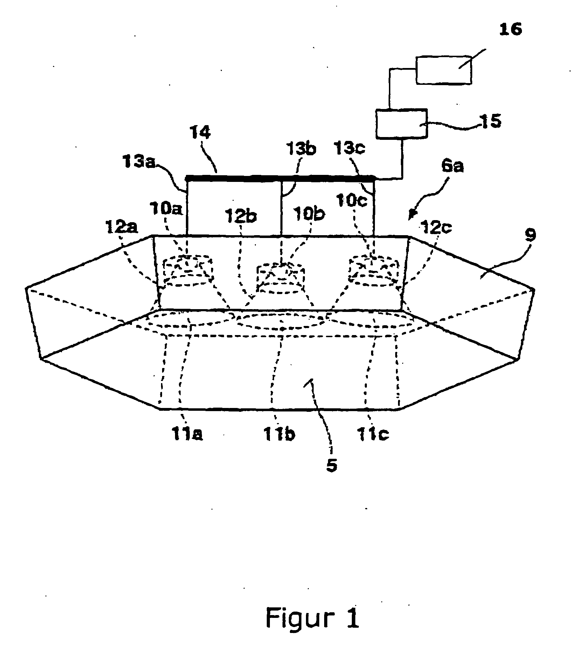

Einzelne zu einer Operationsleuchte kombinierbare Leuchtmodule, beispielsweise ein in der Figur 1 gezeigtes Leuchtmodul 6a, bestehen alle jeweils aus einem Gehäuse 9 mit mechanischen und/oder elektrischen oder elektronischen Verbindungselementen oder Konnektoren zu benachbarten Leuchtmodulen. Die Form der Leuchtmodule ist so gestaltet, dass sie auf einer Kugelfläche mit typischem Radius 1000 mm ohne Zwischenräume angeordnet werden können. Um dies zu erreichen, sind die Leuchtmodule als Sechsecke ausgebildet. In zusammengesetzter Form ergibt sich eine Art Wabenstruktur oder Facettenstruktur. Die der Operationsstelle zugewandte Fläche der Leuchtmodule muss auch nicht zwingend eben ausgebildet sein, sondern kann leicht konkav sein, um die Krümmung der Kugelfläche besser nachzubilden. Die optische Achse des Leuchtmoduls 6a weist im Allgemeinen in den Brennpunkt der Kugelfläche.

Unterschiedliche Lichtfeldformen können durch die Aneinanderreihung von Leuchtmodulen mit verändertem Anstellwinkel erzeugt werden. Dazu können auch Zwischenelemente zum Einsatz kommen. In dem Leuchtmodul 6a sind mehrere, ca. 10 bis 50 LEDs, gleichmäßig verteilt angeordnet, von denen in der Figur 1 lediglich drei gezeigt und mit Bezugszeichen 10a bis 10c bezeichnet sind. Die Schattenbildung wird durch einen flächigen Lichtaustritt optimiert. Hierzu sind jeder der nahezu punktförmig strahlenden LEDs geeignete optische Elemente, z.B. Linsen 11a bis 11c, zugeordnet (gestrichelt angedeutet sind beispielhaft die LED-Lichtstrahlen 12a bis 12c). Die Form der Linsenelemente 11a bis 11c ist so gestaltet, dass sie das Leuchtmodul 6a möglichst bis zum Rand ausfüllen. Die Linsenelemente 11a bis 11c können zudem über eine Streustruktur zur Vergleichmäßigung des Lichtfeldes verfügen. Die Unterseite 5 der Leuchtmodule kann durch eine transparente Scheibe abgedeckt sein.

Die einzelnen Leuchtmodule 6a bilden zusammen eine Lichtquelle mit einer Farbtemperatur von ca. 4.500 K sowie eines Farbwiedergabeindexes Ra > 93, um eine natürliche Farbdarstellung, beispielsweise des zu operierenden Gewebes, zu erreichen. Deshalb kommen nicht nur LEDs 10a zum Einsatz, welche weißes Licht erzeugen, sondern auch LEDs 10b, welche farbiges Licht einer ersten Farbe erzeugen, und LEDs 10c, welche farbiges Licht einer zweiten Farbe erzeugen. Durch Zumischung farbiger Lichtanteile, wie z.B. Cyan und Blau, wird ein Einbruch im Spektrum, wie bei einer Anordnung mit rein weißen LEDs, teilweise kompensiert. Ferner können gezielt Farbmischungen erzeugt werden, die die Sicht des Operateurs verbessern. Bei konstanter Helligkeit der weißen LEDs können durch ausschließliche stufenlose Dimmung der Intensität der farbigen LEDs die Farbtemperatur und Farbwiedergabe des durch das Gesamtmodul, bestehend aus allen einzelnen Leuchtmodulen (Gesamtlichtquelle), erzeugten Mischlichts variabel eingestellt werden. Die Lichtstromintensität der LEDs 10a bis 10c kann stufenlos verändert werden. Anzustreben wäre ferner das Konstanthalten der Gesamtbeleuchtungsstärke durch abgestimmte Intensitätsregelung aller LEDs.

Die LEDs 10a bis 10c sind über Stromleitungen 13a bis 13c mit einer im Leuchtmodul 6a angeordneten Leiterplatte 14 und mit einer zentralen Steuerung 15 verbunden, welche eine elektrische Dimmung des Lichtstroms der LEDs ermöglicht und mithilfe eines Bedienelements 16 betätigt werden kann.

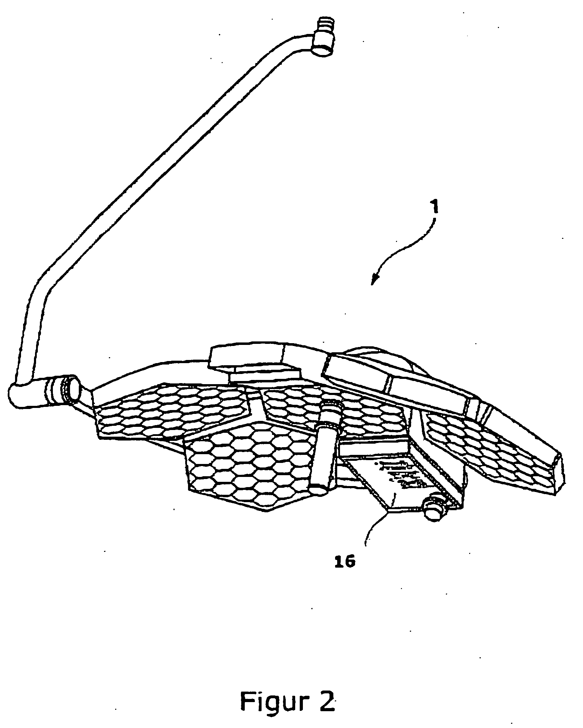

Die variabel steuerbare Operationsleuchte (siehe Figur 2) besteht aus einzelnen Leuchtmodulen, beispielsweise drei oder fünf, die jeweils mit ca. 35 verschiedenfarbigen LEDs als Leuchtmittel ausgestattet sind. Jede einzelne LED mit ihrem optischen System und somit auch jedes Leuchtmodul können das gesamte Leuchtfeld ausleuchten. Durch eine unterschiedliche Ansteuerung der LEDs können verschiedene Leuchtintensitäten und Farbtemperaturen der Operationsleuchte eingestellt werden. Hierbei ist entscheidend, dass die einzelnen Leuchtmodule einen gleichen optischen Eindruck bezüglich der Helligkeit und der Farbtemperatur im Leuchtfeld erzielen. Die LEDs werden erfindungsgemäß gruppenweise zusammengefasst, wobei jede Gruppe einzeln angesteuert werden kann. Kriterien für die Gruppierung sind die Farbe der LEDs und, bei einer Überschreitung der maximalen Strombelastbarkeit der Steuerung, die Anzahl der LEDs. Die einzelnen Gruppen können entweder in Modulen oder anderen Kombinationen zusammengefasst werden. Mit Hilfe der zentralen Steuerung 16, an der vom Bediener die gewünschte Farbtemperatur, Leuchtintensität und Verteilung des austretenden Lichts ausgewählt wird, werden die Gruppen dann angesteuert.

Jedes Modul oder jede Kombination von einzelnen Gruppen von LEDs werden bezüglich der Leuchtintensität und Farbtemperatur vermessen und auf entsprechende Sollwerte kalibriert. Hierbei wird auch der Effekt kompensiert, dass die LEDs bei einer Dimmung durch Reduzierung des Stromflusses (lineare Stromregelung) ihre Farbtemperatur verändern. Ferner weisen die LEDs typischerweise große Fertigungsstreuungen bezüglich der ausgestrahlten Farbtemperatur und Leuchtintensität auf. Aus diesem Grund ist es erforderlich, die Leistungswerte für die einzelnen Gruppen für jedes Modul oder Kombination messtechnisch zu bestimmen, um die erforderlichen Sollwerte zu erreichen. Diese sich aus dem Erreichen der Sollwerte ergebenden - d.h. zum Erreichen der Sollwerte sind bestimmte Leistungswerte jeder Gruppe von Leuchtmitteln erforderlich - Leistungswerte werden für das Modul oder die Kombination der einzelnen Gruppen gespeichert. Die Leistungswerte geben an mit welcher Leistung jede einzelne Gruppe angesteuert werden muss, um die eingestellten Sollwerte zu erreichen. Dieser Leistungswert kann ein Stromwert (lineare Stromregelung) oder eine Impulsfolge für eine Pulsweitenmodulation für den Versorgungsstrom der LEDs sein. Die Kombination der Einstellung für die einzelnen Gruppen ergibt dann die gewünschten Einstellungen für die Sollwerte Farbtemperatur, Intensität und Verteilung. Es besteht auch die Möglichkeit, die Leistungswerte nicht abzuspeichern, sondern während des Betriebs die Farbtemperatur und die Intensität des austretenden Lichtes zu messen und damit die Parameter zu regeln.

Die Leistungswerte können in der zentralen Steuerung gespeichert werden. Vorteilhaft ist es aber, diese Werte dem jeweiligen Modul oder Kombination von Gruppen räumlich oder funktional zuzuordnen. Dies kann beispielsweise auf der Leiterplatte 14 im Modul realisiert werden, auf der Steckverbinder zur Verteilung der elektrischen Versorgung angebracht sind, Hier kann ein Speicherbaustein, z.B. ein EEPROM (Electrically Erasable Programmable Read-Only Memory) oder ein Flash-Speicher aufgebracht werden, auf dem die Werte für dieses Modul oder Zusammenfassung von Gruppen gespeichert sind. Bei der Inbetriebnahme der Operationsleuchte werden diese Werte dann in die zentrale Steuerung übertragen und das Modul oder die Kombination mit ihren kalibrierten Werten betrieben.

Weiterhin kann auf der Leiterplatte im Modul ein Temperatursensor aufgebracht sein oder angeschlossen werden, der die Temperatur im Gehäuse misst und bei einer Temperatur, die oberhalb der als zulässig definierten Temperatur liegt, die Leuchtintensität herunterregelt und somit die Temperatur wieder senkt.

Es wird eine Grundeinstellung einer Farbtemperatur von 4.500 K vorgegeben, welche beim Einschalten der Operationsleuchte automatisch erzeugt wird.

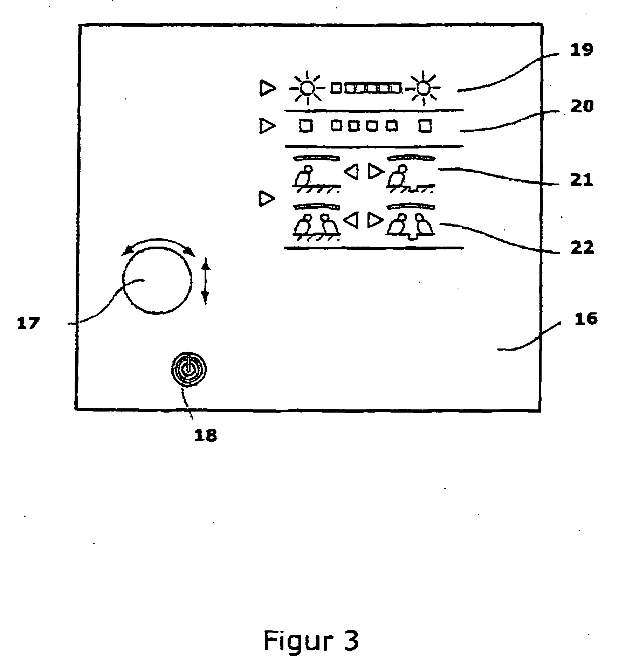

Das Bedienelement 16 weist gemäß Figur 3 einen abnehmbaren und sterilisierbaren Tast-/Drehschalter 17 auf, welcher sowohl beim Drücken als auch bei seiner Verdrehung Impulse an die Steuerung der Operationsleuchte gibt. Beim Drücken des Schalters 17 werden die verschiedenen Betriebszustände der Operationsleuchte nacheinander durchgeschaltet. Hierbei handelt es sich um die Funktionen:
- Ein /Aus (vollständige Abschaltung oder Standby-Zustand)
- Lichtintensität (Helligkeit)
- Farbtemperatur
- Beleuchtungssituation (Auswahl der Intensitätsverteilung des Lichtaustritts)
- optional: Kameraansteuerung (Orientierung, Zoom)

Das definierte stufenweise Verdrehen des Schalters 17 wird durch Raststellungen erleichtert. Dadurch werden innerhalb der Betriebszustände die Betriebsparameter verändert und können an der Bedieneinrichtung 16 angezeigt werden. Die folgenden Parameter sind in einer Steuerung abgespeichert:

| | |
|---|---|
| Lichtintensität: | z.B. Endo (10%) / 50% / 60% / 70% / 80% / 90%/ 100% |
| Farbtemperatur: | z.B. 3 500 K / 4 000 K / 4 500 K / 5 000 K |
| Beleuchtungssituation: | z.B. 1 Operateur / 2 Operateure / großflächige Wunde / tiefe, enge Wunde. |

Das Aus- bzw. Einschalten am sterilen Schalter 17 führt in oder aus einem Standby-Modus. Die Betriebsparameter werden beim Ausschalten abgespeichert und können weiterhin angezeigt werden. Beim Einschalten ist die Operationsleuchte in dem Betriebszustand mit den abgespeicherten, letzten Parametern.

Die Bedieneinrichtung 16 umfasst neben dem Schalter 17 einen weiteren Schalter 18, mit dessen Hilfe die Operationsleuchte vollständig aus- bzw. eingeschaltet werden kann. Beim Einschalten befindet sich die Operationsleuchte dann in einem Zustand mit vordefinierten Parametern (Grundstellung). Die Bedieneinrichtung 16 weist eine Anzeige 19 mit mehreren LEDs zur Darstellung der Intensität der eingestellten Helligkeit der Operationsleuchte, eine Anzeige 20 mit mehreren LEDs zur Darstellung der Intensität der eingestellten Farbtemperatur, eine Anzeige 21 zur Darstellung der Einstellung der Operationsleuchte bezüglich tiefer oder nicht tiefer Wunden und eine Anzeige 22 zur Darstellung der Einstellung der Operationsleuchte bezüglich eines oder mehrerer Operateure innerhalb des Leuchtfelds.

Beispielsweise kann die Ansteuerung folgendermaßen durchgeführt werden:

| | | | Modul 1 | | | | ... | Modul n | | | |
|---|---|---|---|---|---|---|---|---|---|---|---|
| Situation (Verteilung) | Farb-temperatur | Leucht-intensität | ww | kw | bl | cn | | ww | kw | bl | cn |
| S1 | 3500 K | 50% | 60 | 20 | 0 | 0 | ... | 59 | 22 | 0 | 0 |
| S1 | 4000 K | 50 % | 55 | 25 | 20 | 20 | ... | 53 | 27 | 22 | 19 |
| . | . | . | . | . | . | . | ... | . | . | . | . |
| S1 | 5500 K | 100% | 95 | 98 | 85 | 83 | ... | 96 | 99 | 83 | 84 |
| . | . | . | . | . | . | . | ... | . | . | . | . |
| . | . | . | . | . | . | . | ... | . | . | . | . |
| . | . | . | . | . | . | . | ... | . | . | . | . |
| S4 | 5500 K | 100 % | 102 | 85 | 80 | 78 | ... | 0 | 0 | 0 | 0 |

Es bedeuten:
ww = warmweiß;
kw = kaltweiß;
bl = blau;
cn = cyan.

## Patentansprüche

1. Operationsleuchte (1) mit mindestens einem weißen (10a) und mehreren farbigen (10b, 10c) Leuchtmittein zur Ausleuchtung der Operationsstelle und mit einer Steuerung (15) für die Leuchtmittel (10a, 10b, 10c), wobei Mittel zur Speicherung von Leistungswerten für die Leuchtmittel (10a, 10b, 10c) vorgesehen sind und die Steuerung (15) zur Ansteuerung einzelner mehrere Leuchtmittel (10a, 10b, 10c) umfassender Gruppen ausgebildet ist und das Kriterium für die Gruppierung die Farbe der LEDs ist, wobei die Leuchtmittel (10a, 10b, 10c) zu mehreren Modulen (6a) oder Kombinationen zusammengefasst sind, **dadurch gekennzeichnet, dass** iedem Modul (6a) oder Kombination ein Mittel zur Speicherung zugeordnet ist, und die Operationsleuchte (1) so ausgebildet ist, dass diese Leistungswerte in eine zentrale Steuerung übertragen werden.

2. Operationsleuchte nach Anspruch 1, **dadurch gekennzeichnet, dass** die Leitungswerte in Abhängigkeit von Sollwerten bestimmbar sind.

3. Operationsleuchte nach Anspruch 2, **dadurch gekennzeichnet, dass** ein Sollwert die Farbtemperatur der Operationsleuchte (1) ist.

4. Operationsleuchte nach Anspruch 2, **dadurch gekennzeichnet, dass** ein Sollwert die Leuchtintensität der Operationsleuchte (1) ist.

5. Operationsleuchte nach Anspruch 2, **dadurch gekennzeichnet, dass** ein Sollwert die Verteilung der Lichtintensität über die Austrittsfläche (5) ist.

6. Operationsleuchte nach Anspruch 1, **dadurch gekennzeichnet, dass** ein Leistungswert die Stromstärke ist.

7. Operationsleuchte nach Anspruch 1, **dadurch gekennzeichnet, dass** ein Leistungswert eine Impulsfolge für die Pulsweitenmodulation ist.

8. Operationsleuchte nach Anspruch 1, **dadurch gekennzeichnet, dass** Mittel vorgesehen sind, die die Zuordnungen vom Speichermittel zur Steuerung übertragen.

9. Operationsleuchte nach Anspruch 1, **dadurch gekennzeichnet, dass** das Mittel zur Speicherung ein EEPROM ist.

## Claims

1. Surgical lamp (1) with at least one white (10a) and several coloured (10b, 10c) illuminants for illuminating the operating site and with a controller (15) for the illuminants (10a, 10b, 10c), wherein means for storing of power values for the illuminants (10a, 10b, 10c) are provided and the controller (15) is designed for controlling groups comprising several individual illuminants (10a, 10b, 10c) and the criterion for assorting is the colour of the LEDs, wherein the illuminants (10a, 10b, 10c) are embraced to several modules (6a) or combinations, **characterized in that** a storing means is assigned to each module (6a) or combination and the surgical lamp (1) is designed such that these power values are transmitted to a central controller.

2. Surgical lamp according to claim 1, **characterized in that** the power values are determinable based on desired values.

3. Surgical lamp according to claim 2, **characterized in that** one desired value is the colour temperature of the surgical lamp (1).

4. Surgical lamp according to claim 2, **characterized in that** one desired value is the illuminating intensity of the surgical lamp (1).

5. Surgical lamp according to claim 2, **characterized in that** one desired value is the distribution of the light intensity over the emitting surface (5).

6. Surgical lamp according to claim 1, **characterized in that** one power value is the electric current.

7. Surgical lamp according to claim 1, **characterized in that** one power value is a pulse sequence for the pulse width modulation.

8. Surgical lamp according to claim 1, **characterized in that** means are provided that transmit the assignment of the storing means to the controller.

9. Surgical lamp according to claim 1, **characterized in that** the storing means is an EEPROM.

## Revendications

1. Lampe chirurgicale (1) avec au moins un moyen d'éclairage blanc (10a) et plusieurs moyens d'éclairage en couleur (10b, 10c), pour éclairer le site chirurgical et avec une commande (15) pour les moyens d'éclairage (10a, 10b, 10c), des moyens de stockage en mémoire de valeurs de puissance pour les moyens d'éclairage (10a, 10b, 10c) étant prévus, et la commande (15) étant réalisée pour commander des groupes individuels comprenant une pluralité de moyens d'éclairage (10a, 10b, 10c), et le critère de groupage étant la couleur des LEDs, les moyens d'éclairage (10a, 10b, 10c) étant regroupés en plusieurs modules (6a) ou combinaisons, **caractérisée en ce qu'**à chaque module (6a) ou combinaison est associé un moyen de stockage en mémoire, et la lampe chirurgicale (1) étant réalisée de manière que ces valeurs de puissance soient transmises dans une commande centrale.

2. Lampe chirurgicale selon la revendication 1, **caractérisée en ce que** les valeurs de puissance sont susceptibles d'être déterminées en fonction de valeurs de consigne.

3. Lampe chirurgicale selon la revendication 2, **caractérisée en ce qu'**une valeur de consigne est la température de couleur de la lampe chirurgicale (1).

4. Lampe chirurgicale selon la revendication 2, **caractérisée en ce qu'**une valeur de consigne est l'intensité lumineuse de la lampe chirurgicale (1).

5. Lampe chirurgicale selon la revendication 2, **caractérisée en ce qu'**une valeur de consigne est la répartition de l'intensité lumineuse sur la surface de sortie (5).

6. Lampe chirurgicale selon la revendication 1, **caractérisée en ce qu'**une valeur de puissance est l'intensité du courant électrique.

7. Lampe chirurgicale selon la revendication 1, **caractérisée en ce qu'**une valeur de puissance est une succession d'impulsions pour la modulation de longueur des pulsations.

8. Lampe chirurgicale selon la revendication 1, **caractérisée en ce que** des moyens sont prévus, transmettant les associations entre moyens de stockage en mémoire et commande.

9. Lampe chirurgicale selon la revendication 1, **caractérisée en ce que** le moyen de stockage en mémoire est une EEPROM.
